# EUROPEAN PATENT APPLICATION

(11) **EP 3 954 773 A1**
(43) Date of publication of application: **16.02.2022**
(21) Application number: 20190779.7
(22) Date of filing: 12.08.2020
(51) Int. Cl.: C12N 15/113, A61K 31/7088

(54) **COMPOSITIONS AND METHODS FOR THE TREATMENT OF DISEASES BY ENHANCING ARGINASE 2 IN MACROPHAGES**

(71) Applicant: Royal College of Surgeons in Ireland, Dublin 2 (IE)
(72) Inventor: McCoy, Claire, Dublin 2, D2 (IE); Santi, Chiara, Dublin 2, D2 (IE); Dowling, Jennifer, Dublin 2, D2 (IE); Cryan, Sally-Ann, Dublin 2, D2 (IE); Afzal, Remsha, Dublin 2, D2 (IE); Nally, Frances, Dublin 2, D2 (IE); Duffy, Connor, Dublin 2, D2 (IE)
(74) Representative: Murgitroyd & Company

(57) **Abstract**

Target site blockers and their use in enhancing Arginase 2 in macrophages to maintain macrophages in an anti-inflammatory and tissue repair phenotype are disclosed herein. Further disclosed are compositions and the use of the compositions for the treatment and/or prophylaxis of diseases mediated by macrophages such as inflammatory diseases, autoimmune diseases, neurological diseases or reparative diseases.

SEQUENCE LISTING

<110>  Royal College of Surgeons in Ireland

<120>  COMPOSITIONS AND METHODS FOR THE TREATMENT OF DISEASES BY
       ENHANCING ARGINASE 2 IN MACROPHAGES

<130>  224988.EP.01

<160>  43

<170>  PatentIn version 3.5

<210>  1
<211>  785
<212>  DNA
<213>  Homo sapiens

<400>  1
gagacactgt gcactgacat gtttcacaac aggcattcca gaattatgag gcattgaggg      60

gatagatgaa tactaaatgg ttgtctgggt caatactgcc ttaatgagaa catttacaca     120

ttctcacaat tgtaaagttt cccctctatt ttggtgacca atactactgt aaatgtattt     180

ggttttttgc agttcacagg gtattaatat gctacagtac tatgtaaatt taaagaagtc     240

ataaacagca tttattacct tggtatatca tactggtctt gttgctgttg ttccttcaca     300

tttaagtggt ttttcatctt tcctccctcc tcccacagcc tggctataca gtgcatcctt     360

gaactgtcag cccacagcag caatatgctt attctatcca catccctaac atcatgcatt     420

cacaaggtca aagttctggt ccacaaaccc ttccctatag aagttcaatg gctgcgaaag     480

aatttgtagt aaaccaggcc tcccaggatg gcgagctcca gtaagatgat aatggaaagc     540

agcagcttgt tggttgtcac tctacaaaga gaagcaaagt ggggagtagt cagaagtttg     600

gataaccttc cttctaaaca ttttggggtt agacctggga ccacggctgg atactctgag     660

gctgtatgtt tgatcacaca gccacttagc aggaagtact cataaggttc tttagctgtc     720

acttagggat aacactgtct acctcacaga aatgttaaac tgagacaata aaaaccaaag     780

cataa                                                                 785

<210>  2
<211>  37
<212>  DNA
<213>  Homo sapiens

<400>  2
gtttcacaac aggcattcca gaattatgag gcattga                               37

<210>  3
<211>  37
<212>  DNA
<213>  Homo sapiens

<400>  3

attttggtga ccaatactac tgtaaatgta tttggtt                                37

<210>  4
<211>  37
<212>  DNA
<213>  Homo sapiens

<400>  4
ggttttttgc agttcacagg gtattaatat gctacag                                37

<210>  5
<211>  37
<212>  DNA
<213>  Homo sapiens

<400>  5
ggtattaata tgctacagta ctatgtaaat ttaaaga                                37

<210>  6
<211>  37
<212>  DNA
<213>  Homo sapiens

<400>  6
cataaacagc atttattacc ttggtatatc atactgg                                37

<210>  7
<211>  37
<212>  DNA
<213>  Homo sapiens

<400>  7
gtcttgttgc tgttgttcct tcacatttaa gtggttt                                37

<210>  8
<211>  37
<212>  DNA
<213>  Homo sapiens

<400>  8
gttctggtcc acaaaccctt ccctatagaa gttcaat                                37

<210>  9
<211>  37
<212>  DNA
<213>  Homo sapiens

<400>  9
tagggataac actgtctacc tcacagaaat gttaaac                                37

<210>  10
<211>  37
<212>  DNA
<213>  Homo sapiens

<400>  10

gggataacac tgtctacctc acagaaatgt taaactg                                37

<210>  11
<211>  27
<212>  DNA
<213>  Homo sapiens

<400>  11
actgagacaa taaaaaccaa agcataa                                          27

<210>  12
<211>  37
<212>  DNA
<213>  Homo sapiens

<400>  12
cacaacaggc attccagaat tatgaggcat tgagggg                                37

<210>  13
<211>  37
<212>  DNA
<213>  Homo sapiens

<400>  13
ctgtcagccc acagcagcaa tatgcttatt ctatcca                                37

<210>  14
<211>  16
<212>  DNA
<213>  Artificial Sequence

<220>
<223>  Arg2-TSB

<400>  14
gtaatgctgt tgtgaa                                                      16

<210>  15
<211>  15
<212>  DNA
<213>  Artificial Sequence

<220>
<223>  NC-TSB

<400>  15
acgtctatac gccca                                                       15

<210>  16
<211>  41
<212>  DNA
<213>  Artificial Sequence

<220>
<223>  Arg2_clon_F

<400>  16

aacgagctcg ctagcctcga ggaaatactg tactctggca c 41

<210> 17
<211> 45
<212> DNA
<213> Artificial Sequence

<220>
<223> Arg2_clon_R

<400> 17
caggtcgact ctagactcga gtatgatata ctaaggtaat aaatg 45

<210> 18
<211> 48
<212> DNA
<213> Artificial Sequence

<220>
<223> Arg2_TSB_MUT_F

<400> 18
ctctggcacc tttcacaaca gctaatcaga gttgcaaggc attcgaag 48

<210> 19
<211> 48
<212> DNA
<213> Artificial Sequence

<220>
<223> Arg2_TSB_MUT_R

<400> 19
cttcgaatgc cttgcaactc tgattagctg ttgtgaaagg tgccagag 48

<210> 20
<211> 20
<212> DNA
<213> Artificial Sequence

<220>
<223> ASO_Arg2_wt

<400> 20
cacctttcac aacagcatta 20

<210> 21
<211> 20
<212> DNA
<213> Artificial Sequence

<220>
<223> ASO_Arg2_mut

<400> 21
cacctttcac aacagctaat 20

<210>  22
<211>  20
<212>  DNA
<213>  Artificial Sequence

<220>
<223>  pmir_seq_F

<400>  22
gtggtgttgt gttcgtggac                                                    20


<210>  23
<211>  20
<212>  DNA
<213>  Artificial Sequence

<220>
<223>  pmir_seq_R

<400>  23
cagccaactc agcttccttt                                                    20


<210>  24
<211>  20
<212>  DNA
<213>  Artificial Sequence

<220>
<223>  Primers for Arg2 - Forward

<400>  24
ggatccagaa ggtgatggaa                                                    20


<210>  25
<211>  20
<212>  DNA
<213>  Artificial Sequence

<220>
<223>  Primers for Arg2 - Reverse

<400>  25
agagctgaca gcaaccctgt                                                    20


<210>  26
<211>  20
<212>  DNA
<213>  Artificial Sequence

<220>
<223>  Rplp0: forward

<400>  26
ggaccgcctg gttctcctat                                                    20


<210>  27
<211>  20
<212>  DNA

<213>  Artificial Sequence

<220>
<223>  Rplp0: reverse

<400>  27
acgatgtcac tccaacgagg                                                    20


<210>  28
<211>  23
<212>  DNA
<213>  Artificial Sequence

<220>
<223>  Tbp: forward

<400>  28
gaattgtacc gcagcttcaa aat                                                23


<210>  29
<211>  20
<212>  DNA
<213>  Artificial Sequence

<220>
<223>  Tbp: Reverse

<400>  29
cagttgtccg tggctctctt                                                    20


<210>  30
<211>  21
<212>  DNA
<213>  Artificial Sequence

<220>
<223>  hsa-miR-1299 TSB

<400>  30
ttctggaatg cctgttgtga a                                                  21


<210>  31
<211>  17
<212>  DNA
<213>  Artificial Sequence

<220>
<223>  hsa-miR-199a, hsa-miR-199b TSB

<400>  31
tacagtagta ttggtca                                                       17


<210>  32
<211>  17
<212>  DNA
<213>  Artificial Sequence

<220>

<223>  hsa-miR-10b, has-miR-10a TSB

<400>  32
ataccctgtg aactgca                                                    17


<210>  33
<211>  17
<212>  DNA
<213>  Artificial Sequence

<220>
<223>  hsa-miR-1278 TSB

<400>  33
tagtactgta gcatatt                                                    17


<210>  34
<211>  20
<212>  DNA
<213>  Artificial Sequence

<220>
<223>  hsa-miR-570 TSB

<400>  34
caaggtaata aatgctgttt                                                 20


<210>  35
<211>  18
<212>  DNA
<213>  Artificial Sequence

<220>
<223>  hsa-miR-1252 TSB

<400>  35
tgaaggaaca acagcaac                                                   18


<210>  36
<211>  19
<212>  DNA
<213>  Artificial Sequence

<220>
<223>  hsa-miR-3202 tsb

<400>  36
gggaagggtt tgtggacca                                                  19


<210>  37
<211>  17
<212>  DNA
<213>  Artificial Sequence

<220>
<223>  hsa-let-7a, -7b, -7c, -7e, -7f, -7g, -7i, hsa-miR-98 TSB

<400>  37

gtgaggtaga cagtgtt                                                    17

<210>  38
<211>  18
<212>  DNA
<213>  Artificial Sequence

<220>
<223>  hsa-miR-1294 TSB

<400>  38
tgtgaggtag acagtgtt                                                   18

<210>  39
<211>  17
<212>  DNA
<213>  Artificial Sequence

<220>
<223>  hsa-miR-9 TSB

<400>  39
gctttggttt ttattgt                                                    17

<210>  40
<211>  20
<212>  DNA
<213>  Artificial Sequence

<220>
<223>  hsa-miR-155 TSB

<400>  40
cataattctg gaatgcctgt                                                 20

<210>  41
<211>  18
<212>  DNA
<213>  Artificial Sequence

<220>
<223>  hsa-miR-155

<400>  41
atattgctgc tgtgggct                                                   18

<210>  42
<211>  24
<212>  RNA
<213>  Mus musculus

<400>  42
uggcaccuuu cacaacagca uuac                                            24

<210>  43
<211>  23
<212>  RNA

<213>  Mus musculus

<400>  43
uuaaugcuaa uugugauagg ggu                                              23

## Description

### Field of the invention

The present invention relates to target site blockers and their use in enhancing Arginase 2 in macrophages to maintain macrophages in an anti-inflammatory and tissue repair phenotype. The invention further extends to compositions and the use of the compositions of the invention for the treatment and/or prophylaxis of diseases mediated by macrophages such as inflammatory diseases, autoimmune diseases, neurological diseases or reparative diseases.

### Background to the Invention

Macrophages are a sub-type of immune cells that play key roles in the pathogenesis and regulation of the inflammatory reaction. Macrophages are critical for fighting infection in the body through the release of inflammatory and toxic mediators. When macrophages are prone to releasing pro-inflammatory cytokines, they contribute to the development of inflammation which is needed to clear infection and are classified as M1 macrophages. However, if inflammation persists, they can also become destructive, leading to the severe tissue damage observed in variety of inflammatory and autoimmune diseases such as colitis, rheumatoid arthritis (RA) and multiple sclerosis (MS). On the other hand, macrophages can also adopt a M2 phenotype when they produce anti-inflammatory mediators and potentiate cell proliferation, tissue repair and the healing process. Anti-inflammatory stimuli including IL-10, IL-4, IL-13, glucocorticoids and M-CSF are M2 inducers. Therefore, it is desirable to reprogram inflammatory macrophages from an M1 state into an anti-inflammatory M2 state as a therapeutic avenue to control inflammatory and autoimmune disease and promote repair.

Arginase 2 (Arg2) is one of two arginase isoforms. In humans, Arg2 is encoded by the gene ARG2. However, information regarding the function of Arg2 in immune cells is limited. It is known that Arg2 is expressed in macrophages and nervous tissue. It is a mitochondrial associated protein. Arg2 is an enzyme involved in L-arginine metabolism, hydrolysing arginine to ornithine and urea. Previously, Arg2 has been shown to account for more than 90% of arginase activity in LPS activated Raw 264.7 macrophages. It was also identified as a gene upregulated by IL-10 in M(LPS) macrophages by an Affymetrix array, and was confirmed as a miRNA (miR-155) target in dendritic cells (DC). The inventors have recently shown that Arg2 is strongly upregulated and critical for arginine metabolism in IL-10 stimulated inflammatory macrophages. IL-10 is intricately involved in regulating arginine metabolism in macrophages, acting as a regulator for the M2 state in macrophages.

MicroRNAs (miRNAs) are evolutionarily conserved non-coding RNAs that negatively regulate gene expression by repressing translation or decreasing mRNA stability. A miRNA is a small non-coding RNA molecule (containing about 22 nucleotides) found in plants, animals and some viruses, that functions in RNA silencing and post-transcriptional regulation of gene expression. MiR-155 is a microRNA that in humans is encoded by the *MIR155* host gene or *MIR155HG.* MiR-155 plays a role in various physiological and pathological processes. Exogenous molecular control *in vivo* of miR-155 expression may affect malignant growth, viral infections, and the progression of cardiovascular diseases. Studies have indicated that there is an intimate relationship between inflammation, innate immunity and miR-155 expression. MiR-155 inhibition with an anti-miR-155 has shown encouraging results in terms of disease progression of some inflammatory diseases in animal models.

There is currently no cure for most inflammatory or autoimmune diseases mediated by macrophages such as colitis, rheumatoid arthritis (RA) and multiple sclerosis (MS). The important role of immune cells in the initiation and progression of these diseases has been clearly established and has deeply influenced therapeutic approaches. The currently approved drugs for colitis, RA and MS are designed to limit inflammation through the use of non-steroidal anti-inflammatory drugs (NSAIDS) and steroids, to limit immune cell replication (azathiorprine, cyclosporing, methotrexate), to block immune cell infiltration into the affected tissue (Natalizumab, Fingolimod, Mitoxantrone) or to reduce immune cell activity (Interferon-β, Glatiramer acetate, Dimethyl fumarate). Therapeutic approaches also include drugs to deplete immune cells (Rituxumab, Ocrelizumab, Alemtuzumab) or to inhibit inflammatory cytokines such as tumour necrosis factor and interleukin-1 (etanercept, infliximab, anakinra). Although these treatments have shown some efficacy they are characterised by multiple side-effects, including systemic immune suppression which leaves an individual susceptible to opportunistic infections and cancer. Furthermore, while the current treatment regimens limit inflammation in the early stages of disease, the underlying tissue damage continues to progress irrespectively (particularly in RA and MS). Moreover, there are no treatments for the secondary and primary progressive stages of MS.

There are currently no completely effective therapeutic or prophylactic treatments that manipulate macrophages for the treatment of inflammatory, autoimmune, neurological or reparative disorders. There is a significant unmet need to identify novel targets that can both limit inflammation during an immune response and promote repair within the cell in order to provide effective treatments. Thus, there exists a need for a safe, effective treatment for inflammatory, autoimmune, neurological or reparative disorders mediated by macrophages, and in particular for the treatment and/or prophylaxis of multiple sclerosis, rheumatoid arthritis and colitis.

### Summary of Invention

Following extensive experimentation, the present inventors have surprisingly identified a unique mechanism to switch off damaging macrophages and maintain them in an anti-inflammatory and tissue repairing phenotype. The present inventors have identified that enhancing the expression of an enzyme called Arginase 2 (Arg2) is critical for maintaining macrophages in an anti-inflammatory and tissue regenerative state. In particular, the present inventors have unexpectedly identified that the expression of Arg2 can be enhanced by using target site blockers (TSBs) that directly and specifically interfere with or inhibit individual microRNA binding sites within Arg2. Target site blockers are locked-nucleic acid antisense oligonucleotides that specifically compete with miRNAs for binding to individual miRNA recognition elements (MREs) of a target mRNA, hence preventing them from gaining access to those sites. The miRNA-binding sequence in the target is generally referred to as the miRNA recognition element or MRE. A microRNA (for example miR-155) binds its target mRNA (for example on Arg2) through sequence-specific miRNA recognition elements (MREs) within the 3'untranslated region (3'UTR) of Arg2, impeding its translation and leading to low quantity of the protein product.

Suitably an assay, for example a luciferase assay experiment, in which cells co-transfected pmir_Arg2_wt or pmir_Arg2_mut TSB sequences are utilised to determine if there is a decrease in luciferase activity test mRNA binding sites and evaluate the regulation or the miRNA activity (other suitable reporting assays as would be known in the art could also be utilised to test mRNA binding sites and evaluate the regulation or the miRNA activity). Suitably, luciferase activity may be assessed at 24 hours after transfection using Dual-Luciferase Reporter Assay (Promega) or the like.

The present inventors have developed novel nucleic acids specific to the 3'untranslated region of Arg2 that enhance Arg2 in macrophages. These novel nucleic acids act as TSBs to inhibit microRNA binding in Arg2. Specifically, the present inventors have designed TSBs to compete with miR-155-5p (now on referred as miR-155) binding to its specific site within Arginase 2 (Arg2). In particular, the present inventors have designed human TSBs specific to the 3'UTR of Arg2 that binds to the miR-155 target site at MRE positions 39-46 and 379-386. The present inventors have also designed a murine TSB specific to the 3'UTR of Arg2 that binds to the miR-155 target site at MRE position 30-37.
Manipulation of miRNAs has been explored as a potential therapeutic strategy in several diseases. However, very often typical anti-miR strategies are prone to off-target effects, due to the intrinsic ability of miRNAs to regulate multiple mRNAs in different cell types, some of whom might be necessary for physiological homeostasis. The present inventors have overcome this issue by using target site blockers which directly and specifically interfere with individual miRNA within the novel target Arg2, thereby limiting the side effects. In addition, the present inventors have encapsulated or complexed the novel target site blockers in biocompatible nanoparticles, such as, poly(lactic-co-glycolic acid) nanoparticles (PLGA-NPs) for drug delivery to macrophages. The PLGA-NPs are favoured for uptake by macrophages in order to minimise the uptake in other cell types.

This has led to the identification by the inventor of improved therapeutic compositions that have utility in the treatment and/or prophylaxis of an inflammatory, autoimmune, neurological or reparative condition where macrophages play a role, in particular multiple sclerosis (MS), rheumatoid arthritis (RA) and colitis.

Accordingly a first aspect of the present invention provides a target site blocker or combinations of target site blockers for enhancing Arginase 2 expression in macrophages wherein the target site blocker is specific to miRNA binding sites in Arginase 2.

In embodiments, the target site blocker is specific to miRNA binding sites in the 3'untranslated region of Arginase 2.

In embodiments, the target site blocker is specific to a miRNA binding site in Arginase 2 wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites.

In embodiments, the target site blocker is specific to a miR-155 binding site in Arginase 2.

In embodiments, the target site blocker is a nucleic acid molecule. In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof or combinations of said target site blockers.

In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a combination thereof.

In embodiments, the target site blocker is encapsulated or complexed in a biocompatible nanoparticle. In embodiments, the biocompatible nanoparticle is a poly(lactic-co-glycolic acid) nanoparticle (PLGA-NP).

According to a second aspect of the present invention, there is provided a target site blocker for enhancing Arginase 2 expression in macrophages, wherein the target site blocker is specific to miRNA binding sites in Arginase 2, for use in the treatment and/or prophylaxis of conditions mediated by macrophages.

In embodiments, the target site blocker is specific to miRNA binding sites in the 3'untranslated region of Arginase 2.

In embodiments, the target site blocker is specific to a miRNA binding site in Arginase 2 wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites.

In embodiments, the target site blocker is specific to a miR-155 binding site in Arginase 2.

In embodiments, the target site blocker is a nucleic acid molecule. In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof.

In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a combination thereof.

In embodiments, the conditions mediated by macrophages are inflammatory conditions, autoimmune conditions, neurological conditions or reparative conditions.

In embodiments, the inflammatory or autoimmune condition mediated by macrophages is multiple sclerosis, rheumatoid arthritis or colitis.

In embodiments, the neurological condition mediated by macrophages is a neurodegenerative condition selected from the group comprising amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease.

In embodiments, the reparative condition is peripheral nerve repair after injury.

In embodiments, the target site blocker is encapsulated or complexed in a biocompatible nanoparticle. In embodiments, the biocompatible nanoparticle is a poly(lactic-co-glycolic acid) nanoparticle (PLGA-NP).

According to a third aspect of the present invention there is provided a method for the treatment and/or prophylaxis of a condition mediated by macrophages, said method comprising the step of:
(i) administering to a subject in need thereof a therapeutically effective amount of a target site blocker for enhancing Arginase 2 expression in macrophages wherein the target site blocker is specific to miRNA binding sites in Arginase 2.

In embodiments, the target site blocker is specific to miRNA binding sites in the 3'untranslated region of Arginase 2.

In embodiments, the target site blocker is specific to a miRNA binding site in Arginase 2 wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites.

In embodiments, the target site blocker is specific to a miR-155 binding site in Arginase 2.

In embodiments, the target site blocker is a nucleic acid molecule. In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof.

In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a combination thereof.

In embodiments, the conditions mediated by macrophages are inflammatory conditions, autoimmune conditions, neurological conditions or reparative conditions.

In embodiments, the inflammatory or autoimmune condition mediated by macrophages is multiple sclerosis, rheumatoid arthritis or colitis.

In embodiments, the neurological condition mediated by macrophages is a neurodegenerative condition selected from the group comprising amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease.

In embodiments, the reparative condition is peripheral nerve repair after injury.

In embodiments, the target site blocker is encapsulated or complexed in a biocompatible nanoparticle. In embodiments, the biocompatible nanoparticle is a poly(lactic-co-glycolic acid) nanoparticle (PLGA-NP).

According to a fourth aspect of the present invention, there is provided use of a target site blocker for enhancing Arginase 2 expression in macrophages, wherein the target site blocker is specific to miRNA binding sites in Arginase 2, for the treatment and/or prophylaxis of a condition mediated by macrophages.

In embodiments, the target site blocker is specific to miRNA binding sites in the 3'untranslated region of Arginase 2.

In embodiments, the target site blocker is specific to a miRNA binding site in Arginase 2 wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites.

In embodiments, the target site blocker is specific to a miR-155 binding site in Arginase 2.

In embodiments, the target site blocker is a nucleic acid molecule. In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof.

In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a combination thereof.

In embodiments, the conditions mediated by macrophages are inflammatory conditions, autoimmune conditions, neurological conditions or reparative conditions.

In embodiments, the inflammatory or autoimmune condition mediated by macrophages is multiple sclerosis, rheumatoid arthritis or colitis.

In embodiments, the neurological condition mediated by macrophages is a neurodegenerative condition selected from the group comprising amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease.

In embodiments, the reparative condition is peripheral nerve repair after injury.

In embodiments, the target site blocker is encapsulated or complexed in a biocompatible nanoparticle. In embodiments, the biocompatible nanoparticle is a poly(lactic-co-glycolic acid) nanoparticle (PLGA-NP).

According to a fifth aspect of the present invention, there is provided a pharmaceutical composition comprising:
(i) a target site blocker for enhancing Arginase 2 expression in macrophages, wherein the target site blocker is specific to miRNA binding sites in Arginase 2;
(ii) a biocompatible nanoparticle carrier.

In embodiments, the target site blocker is specific to miRNA binding sites in the 3'untranslated region of Arginase 2.

In embodiments, the target site blocker is specific to a miRNA binding site in Arginase 2 wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites.

In embodiments, the target site blocker is specific to a miR-155 binding site in Arginase 2.

In embodiments, the target site blocker is a nucleic acid molecule. In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof.

In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a combination thereof.

In embodiments, the pharmaceutical composition is for use in the treatment and/or prophylaxis of a condition mediated by macrophages. In embodiments, the conditions mediated by macrophages are inflammatory conditions, autoimmune conditions, neurological conditions or reparative conditions.

In embodiments, the inflammatory or autoimmune condition mediated by macrophages is multiple sclerosis, rheumatoid arthritis or colitis.

In embodiments, the neurological condition mediated by macrophages is a neurodegenerative condition selected from the group comprising amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease.

In embodiments, the reparative condition is peripheral nerve repair after injury.

In embodiments, the target site blocker is encapsulated or complexed in the biocompatible nanoparticle or microparticle. In embodiments, the biocompatible nanoparticle or microparticle is a poly(lactic-co-glycolic acid) nanoparticle (PLGA-NP). In embodiments, the biocompatible nanoparticle is a polymer-based nanoparticle. In embodiments, the polymer-based nanoparticle is selected from the group comprising or consisting of Polyethylenimine (PEI) or its copolymers, Poly-L-lysine (PLL) or its copolymers, Polyethylene glycol (PEG) or its copolymers, Polycaprolactone, chitosan, Acetalated dextran or Star-Shaped Poly(l-lysine) Polypeptides. In embodiments, the biocompatible nanoparticle is a Star-Shaped Poly(l-lysine) Polypeptide. In embodiments of the above aspects of the invention, the biocompatible nanoparticle or microparticle is a liposome-based nanoparticle. In embodiments, the liposome-based nanoparticle is Polyethylene glycol (PEG)-liposome. In embodiments of the above aspects of the invention, the biocompatible nanoparticle is an inorganic nanoparticle. In embodiments, the inorganic nanoparticle is selected from the group comprising or consisting of gold, inorganic-organic hybrid or silica

According to a further aspect of the present invention, there is provided use of a target site blocker for enhancing Arginase 2 expression in macrophages, wherein the target site blocker is specific to miRNA binding sites in Arginase 2, in the preparation of a medicament for the treatment and/or prophylaxis of a condition mediated by macrophages.

According to a further aspect of the present invention, there is provided a composition comprising a target site blocker for enhancing Arginase 2 expression in macrophages, wherein the target site blocker is specific to miRNA binding sites in Arginase 2, for use in the treatment and/or prophylaxis of a condition mediated by macrophages.

According to a further aspect of the present invention, there is provided a pharmaceutical composition comprising a target site blocker for enhancing Arginase 2 expression in macrophages, wherein the target site blocker is specific to miRNA binding sites in Arginase 2, for use in the treatment and/or prophylaxis of a condition mediated by macrophages.

According to a further aspect of the present invention, there is provided a method of treating a condition mediated by macrophages in a subject by the administration of a composition comprising a target site blocker which enhances the expression of Arg2 in macrophages, wherein the target site blocker is specific to miRNA binding sites in Arginase 2.

In embodiments of the above aspects of the invention, the target site blocker is specific to miRNA binding sites in the 3'untranslated region of Arginase 2.

In embodiments of the above aspects of the invention, the target site blocker is specific to a miRNA binding site in Arginase 2 wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites.

In embodiments of the above aspects of the invention, the target site blocker is specific to a miR-155 binding site in Arginase 2.

In embodiments of the above aspects of the invention, the target site blocker is a nucleic acid molecule. In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof.

In embodiments, the nucleic acid molecule comprises or consists of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a combination thereof.

In embodiments of the above aspects of the invention, the conditions mediated by macrophages are inflammatory conditions, autoimmune conditions, neurological conditions or reparative conditions.

In embodiments of the above aspects of the invention, the inflammatory or autoimmune condition mediated by macrophages is multiple sclerosis, rheumatoid arthritis or colitis.

In embodiments, the neurological condition mediated by macrophages is a neurodegenerative condition selected from the group comprising amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease.

In embodiments of the above aspects of the invention, the reparative condition is peripheral nerve repair after injury.

In embodiments of the above aspects of the invention, the target site blocker is encapsulated or complexed in a biocompatible nanoparticle. In embodiments of the above aspects of the invention, the biocompatible nanoparticle is a poly(lactic-co-glycolic acid) nanoparticle (PLGA-NP). In embodiments of the above aspects of the invention, the biocompatible nanoparticle is a polymer-based nanoparticle. In embodiments, the polymer-based nanoparticle is selected from the group comprising or consisting of Polyethylenimine (PEI) or its copolymers, Poly-L-lysine (PLL) or its copolymers, Polyethylene glycol (PEG) or its copolymers, Polycaprolactone, chitosan, Acetalated dextran or Star-Shaped Poly(l-lysine) Polypeptides. In embodiments of the above aspects of the invention, the biocompatible nanoparticle is a liposome-based nanoparticle. In embodiments, the liposome-based nanoparticle is Polyethylene glycol (PEG)-liposome. In embodiments of the above aspects of the invention, the biocompatible nanoparticle is an inorganic nanoparticle. In embodiments, the inorganic nanoparticle is selected from the group comprising or consisting of gold, inorganic-organic hybrid or silica.

In embodiments of the above aspects of the invention, enhanced Arg2 expression may encompass enhanced Arg2 mRNA and protein abundance or enhanced Arg2 activity (e.g., enzymatic activity). An enhancement in activity, for example, may be an enhancement relative to levels observed in unmodified macrophages, M1 and M2-like phenotypes. Arg2 increased levels and activity may be assessed by ARG2 gene expression levels (e.g. by qPCR), Arg2 protein abundance (e.g., by quantification with labelled antibodies), or Arg2 enzymatic activity (e.g., by colorimetric assays).

In embodiments of the above aspects of the invention, the target site blocker inhibits the binding of miRNA to Arg2 which means that the target site blocker blocks or inactivates the active binding site of miRNA in Arg 2. In embodiments, the target site blocker blocks or inactivates a miR-155 binding site in the 3'untranslated region of Arg2. In embodiments of the aspects of the invention, the active binding site of miR-155 in human Arg2 is at positions 29-46 and 379-386 in the 3'untranslated region of human Arginase 2. In embodiments of the aspects of the invention, the active binding site of miR-155 in murine Arg2 is at position 30-37 in the 3'untranslated region of murine Arginase 2.

In embodiments of the aspects of the invention, the target site blocker will increase Arg2 expression and restore its physiological levels.

In embodiments of the aspects of the invention, the target site blocker is any target site blocker designed to effectively compete with any endogenous miRNA by hybridizing to the same miRNA recognition element within Arg2 mRNA sequence. In embodiments, the endogenous miRNA is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9. In embodiments, the target site blocker binds to an miRNA recognition element within the Arg2 mRNA sequence at a position selected from the group comprising or consisting of position 36-43, position 39-46, position 163-169, position 196-203, position 215-222, position 255-261, position 291-298, position 448-454, position 739-746, position 741-748, position 774-780 or position 379-386. In embodiments, the target site blocker binds to a binding site comprising or consisting of the nucleotide sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13 or variants or fragments thereof.

In embodiments of the above aspects of the invention, the subject is a human. In embodiments, the subject is a human in need of treatment of an inflammatory, autoimmune, neurological or reparative condition mediated by macrophages. In embodiments, the inflammatory or autoimmune condition mediated by macrophages is multiple sclerosis, rheumatoid arthritis or colitis. In embodiments, the neurological condition mediated by macrophages is a neurodegenerative condition selected from the group comprising amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease.

### Description

The inventors of the present invention have surprisingly discovered that enhancing the expression of an enzyme called Arginase 2 (Arg2) is critical for maintaining macrophages in an anti-inflammatory and tissue regenerative state. The present inventors propose Arginase-2 as a novel anti-inflammatory marker and that enhancing its expression could polarise macrophages towards a regenerative phenotype with potential application in inflammatory, autoimmune, neurological or reparative conditions mediated by macrophages such as multiple sclerosis, rheumatoid arthritis, colitis or peripheral nerve repair after injury. In particular, the present inventors have unexpectedly identified that the expression of Arg2 can be enhanced by using target site blockers (TSBs) that directly and specifically interfere with microRNA binding sites within Arg2, such as miR-155 binding sites.

Recent evidence highlights the importance of mitochondrial bioenergetics and dynamics in regulating macrophage polarisation. It has been previously demonstrated that Arginase 2 (Arg2), one of two arginase isoforms is an interleukin-10 (IL-10) regulated gene localized at mitochondria in inflammatory macrophages and is critical for IL-10 induced restoration of oxidative respiration in these cells. IL-10 radically affects mitochondrial dynamics by promoting a state of 'fusion', which likely facilitates the higher oxidative bioenergetics observed. The present inventors have previously established that Arg2 is essential for activity of succinate dehydrogenase (SDH), a bi-functional enzyme that links the mitochondrial electron transport chain (ETC) and the TCA cycle. Through its regulation of SDH, Arg2 is crucial for unblocking the inflammatory break in the TCA cycle by promoting fumarate and reversing the build-up of inflammatory mediators HIF1α and IL-1β. Without wishing to be bound by theory, the inventors hypothesise that treatment of inflammatory macrophages with IL-10 dramatically alters mitochondrial dynamics to a state of fusion, an effect that has never been observed for any other M2 agonist. Strikingly this is Arg2 dependent requiring both its enzymatic activity and its physical presence at the mitochondria, as the arginase inhibitor nor-Noha or genetic deletion of Arg2 inhibited IL-10 mediated fusion. The present inventors have shown that IL-10 can modulate inflammatory cytokines, NO, mitochondrial dynamics, OxPhos and the TCA cycle via the upregulation of Arg2 at the mitochondria highlighting the extraordinary capacity of this arginase isoform for the resolution of inflammation. Most strikingly, Arg2 is critical for limiting IL-1β production in macrophages. Considering IL-1β is prominent in multiple auto-inflammatory and chronic diseases, the present inventors have uncovered a novel mechanism for therapeutic benefit.

The present inventors have studied the regulation of microRNAs by IL-10 and have previously demonstrated that it acts as a potent inhibitor of miR-155 in inflammatory macrophages. IL-10 inhibited the primary transcript and mature miR-155 in a STAT3 dependent manner. IL-10 could reverse the inhibition of miR-155 target genes such as *ship1,* suggesting that the IL-10/miR-155 axis may be a significant mechanism that harnesses macrophages towards an anti-inflammatory state. In order to illuminate novel IL-10 mediated regulation of metabolic reprogramming, the present inventors used a unique bioinformatics approach that identified Arginase-2 (Arg2) as a potential target.

The present inventors recognise that there is an urgent need to identify novel targets that can both limit inflammation and promote repair within the cell. The present inventors have identified that the specific modulation of macrophages can address this unmet need for the following reasons. Macrophages are the first responders in all inflammatory conditions, releasing potent amounts of inflammatory mediators such as (IL-6, TNF, IL-1) and nitric oxide (NO). Directly targeting these macrophages will limit inflammation quickly and effectively. Inflammatory mediators produced by macrophages are the main source of tissue damage in inflammatory conditions such as acute or chronic inflammatory conditions or viral induced inflammation. Directly targeting these macrophages will limit tissue damage. Enhancing Arginase 2 will skew macrophages from an inflammatory phenotype into an anti-inflammatory and reparative phenotype. Not only will the present invention limit inflammation, but it will also specifically promote repair in the tissue. The inventors have demonstrated that the target site blockers of the present invention dramatically decrease IL-6, a cytokine that is prominent in acute inflammatory conditions such as pneumonia, acute respiratory distress syndrome and Covid-19 related acute respiratory distress syndrome. Covid-19 is an infectious disease caused by severe acute respiratory syndrome coronavirus 2 (SARS-CoV-2).

It still remains elusive how macrophages switch from a pro-inflammatory phenotype to an anti-inflammatory phenotype, however without wishing to be bound by theory, the present inventors propose that enhancing arginase 2 expression is critical for this transition for a variety of reasons. The inventors have demonstrated that arginase 2 is strongly upregulated in anti-inflammatory and reparative macrophages and that arginase 2 is critical for arginine metabolism in anti-inflammatory and reparative macrophages. The inventors show that arginase 2 maintains this phenotype via its localisation at the mitochondria and that arginase 2 is critical for maintaining an anti-inflammatory state by altering mitochondrial morphology. Arginase 2 is critical for maintaining an anti-inflammatory state through its metabolic regulation of oxidative phosphorylation and ATP production and through the production of fumerate, a metabolite critical for the proper functioning of the Kreb's cycle. Arginase 2 is critical for limiting nitric oxide production and IL-1 production in an inflammatory macrophage. The present inventors have demonstrated that they can enhance Arg 2 through the use of target site blockers specific for miRNA binding sites in Arg2.

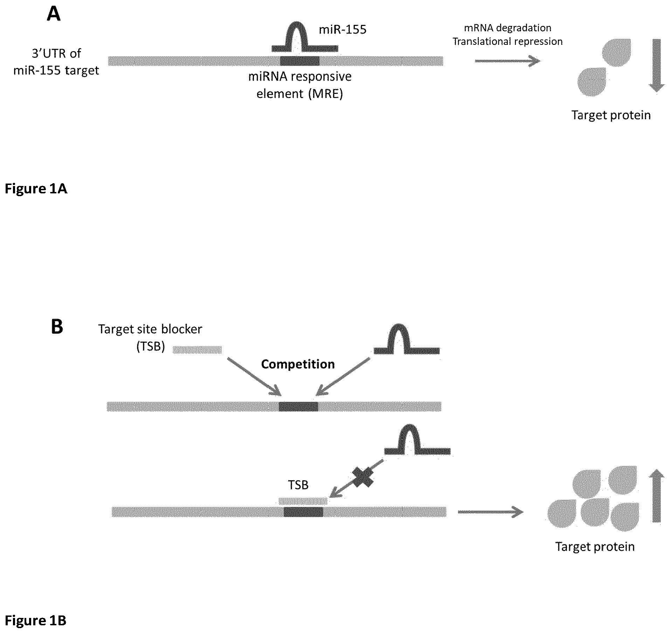

Specifically, the present inventors have developed novel nucleic acids specific to the 3'untranslated region of Arg2 that enhance Arg2 in macrophages. These novel nucleic acids act as target site blockers to inhibit miRNA binding in Arg2, for example miR-155 binding. In particular, the present inventors have developed target site blockers that comprise or consist of the nucleotide sequence of (SEQ ID NOs:30-41) or a functionally active fragment or variant thereof. The present inventors have overcome issues in the prior art by using nucleic acids which directly and specifically interfere with individual miRNA binding sites within the novel target Arg2, thereby limiting the side effects. A microRNA (for example miR-155) binds its target mRNA (for example Arg2) through sequence-specific miRNA responsive elements (MREs) within the 3'untranslated region (3'UTR), impeding its translation and leading to low quantity of the protein product (see Figure 1A). Target Site Blockers are antisense oligonucleotides designed to effectively compete with endogenous miRNA by hybridizing to the same MRE. As a result TSBs will prevent endogenous miRNAs from binding to their MREs thereby increasing the expression of the protein encoded by the targeted mRNA and restoring its physiological levels (see Figure 1B).

Human Arginase 2 has the following sequence (SEQ ID NO:1):

Human Arg2 3'UTR sequence:>hg38_refGene_NM_001172 range=chr14:67650921-
67651705 5'pad=0 3'pad=0 strand=+ repeatMasking=none

Gagacactgtgcactgacatgtttcacaacaggcattccagaattatgag
gcattgaggggatagatgaatactaaatggttgtctgggtcaatactgcc
ttaatgagaacatttacacattctcacaattgtaaagtttcccctctatt
ttggtgaccaatactactgtaaatgtatttggtttttttgcagttcacagg
gtattaatatgctacagtactatgtaaatttaaagaagtcataaacagca
tttattaccttggtatatcatactggtcttgttgctgttgttccttcaca
tttaagtggtttttcatctttcctccctcctcccacagcctggctataca
gtgcatccttgaactgtcagcccacagcagcaatatgcttattctatcca
catccctaacatcatgcattcacaaggtcaaagttctggtccacaaaccc
ttccctatagaagttcaatggctgcgaaagaatttgtagtaaaccaggcc
tcccaggatggcgagctccagtaagatgataatggaaagcagcagcttgt
tggttgtcactctacaaagagaagcaaagtggggagtagtcagaagtttg
gataaccttccttctaaacattttggggttagacctgggaccacggctgg
atactctgaggctgtatgtttgatcacacagccacttagcaggaagtact
cataaggttctttagctgtcacttagggataacactgtctacctcacaga
aatgttaaactgagacaataaaaaccaaagcataa

Although using TSBs directed towards specific miRNA targets, such as miR-155, has been explored in some disorders, for example certain cancers, the present inventors are the first to attempt to specifically block miRNA interaction with Arginase-2 in macrophages as a therapeutic approach for inflammatory, autoimmune and reparative conditions mediated by macrophages. The present inventors are the first to demonstrate a potential effect of the TSBs of the present invention in macrophage polarisation. Table 1 demonstrates sequences and binding sites for the design of the target site blockers of the present invention. The binding sites of each miRNA are underlined in each sequence. Table 1 also demonstrates the miRNA that the target site blockers of the present invention bind to in human Arg2 and the MRE position on the 3'untranslated region of human Arg2 that the target site blockers of the present invention bind to.

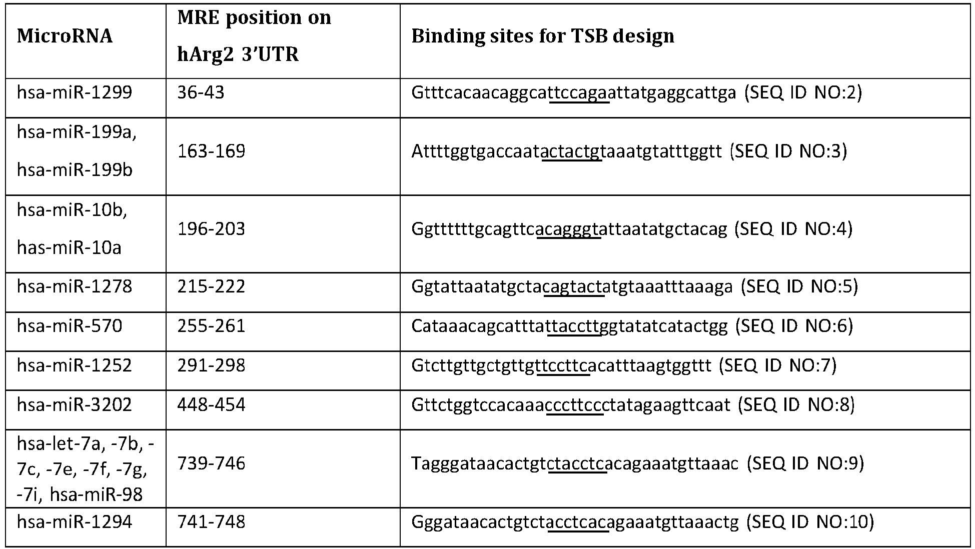

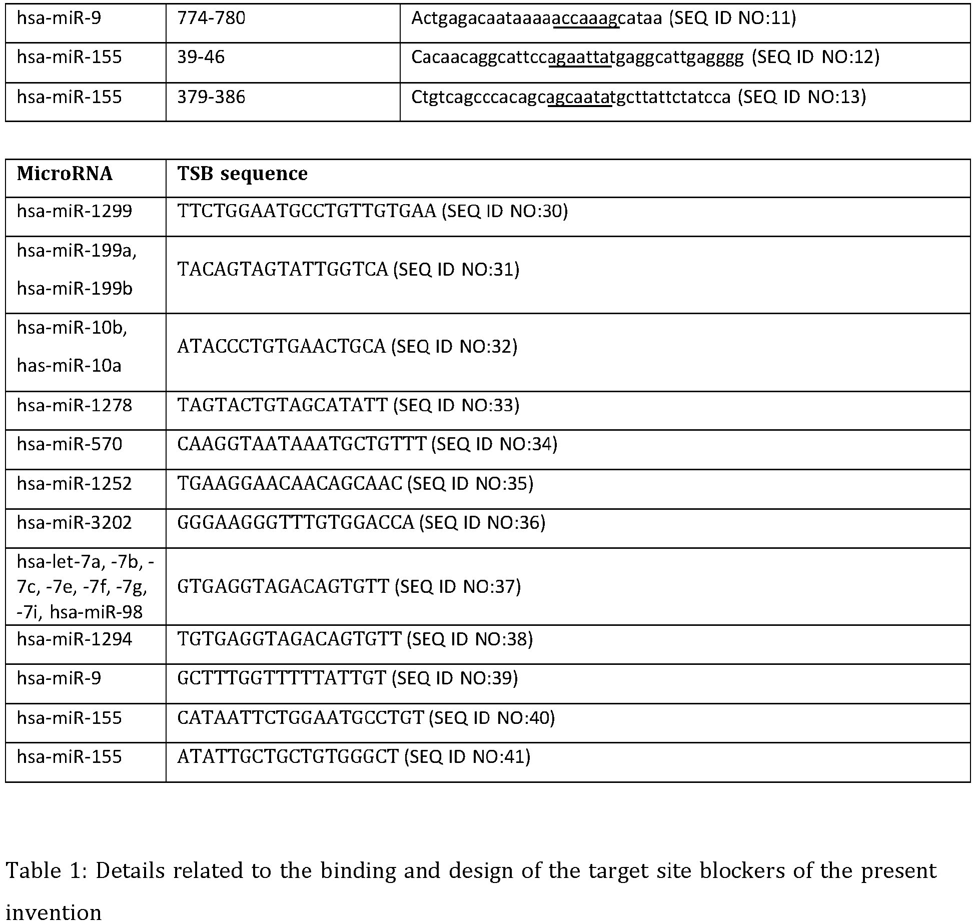

The target site blockers of the present invention are any target site blocker designed to effectively compete with any endogenous miRNA by hybridizing to the same miRNA recognition element within Arg2 mRNA sequence. The endogenous miRNA can be selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9. The target site blocker of the present invention binds to an miRNA recognition element within the Arg2 mRNA sequence at a position selected from the group comprising or consisting of position 36-43, position 39-46, position 163-169, position 196-203, position 215-222, position 255-261, position 291-298, position 448-454, position 739-746, position 741-748, position 774-780 or position 379-386. The target site blockers of the present invention can bind to a binding site comprising or consisting of the nucleotide sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13 or variants or fragments thereof.

In addition, the present inventors have encapsulated or complexed the TSBs of the present invention in biocompatible nanoparticles, such as, poly(lactic-co-glycolic acid) nanoparticles (PLGA-NPs), for specific drug delivery to macrophages. The PLGA-NPs are favourable for uptake by macrophages which minimises the uptake in other cell types. The combination of this RNA-based technology with PLGA nanoparticles has never been attempted before in macrophages and represents a novel delivery strategy which will guarantee the efficient delivery of the therapeutics in *in vivo* experiments.

Other biocompatible nanoparticles can also be used for specific delivery of the novel target site blockers of the present invention. The biocompatible nanoparticle can be a polymer-based nanoparticle such as one selected from the group comprising or consisting of Polyethylenimine (PEI) or its copolymers, Poly-L-lysine (PLL) or its copolymers, Polyethylene glycol (PEG) or its copolymers, Polycaprolactone, chitosan, Acetalated dextran or Star-Shaped Poly(l-lysine) Polypeptides. The biocompatible nanoparticle can be a liposome-based nanoparticle, such as Polyethylene glycol (PEG)-liposome. The biocompatible nanoparticle can be an inorganic nanoparticle such as one selected from the group comprising or consisting of gold, inorganic-organic hybrid or silica.

The present inventors have demonstrated that the *in vivo-ready* TSB-PLGA nanoparticles of the present invention are able to polarise macrophages towards an anti-inflammatory phenotype by increasing the expression of Arginase-2, a novel "M2"-like marker in macrophages as a proof of principle for specific drug delivery. The present inventors have produced an improved therapeutic for inflammatory, autoimmune or reparative conditions mediated by macrophages and in particular for multiple sclerosis.

### Definitions

### Target Site Blockers

Target site blockers (TSBs) are locked-nucleic acid antisense oligonucleotides that specifically compete with miRNAs for the binding to individual miRNA recognition elements (MREs) of a target mRNA, hence preventing them from gaining access to those sites. Target site blockers are high affinity antisense oligonucleotides that enable researchers to study the biological consequences of blocking the microRNA interaction with a specific mRNA. The target site blockers of the present invention inhibit the activity or function of microRNA (miRNA) by fully or partially hybridizing to MREs within the target mRNA thereby repressing the function or activity of the miRNA.

The oligonucleotide of the invention may be of any suitable type. The person skilled in the art can easily provide some modifications that will improve the clinical efficacy of the oligonucleotide. Typically, chemical modifications include backbone modifications, heterocycle modifications, sugar modifications, and conjugations strategies. For example the oligonucleotide may be selected from the group consisting of oligodeoxyribonucleotides, oligoribonucleotides, LNA, oligonucleotide, morpholinos, tricyclo-DNA-antisense oligonucleotides (ASOs), U7- or U1-mediated ASOs or conjugate products thereof such as peptide-conjugated or nanoparticle-complexed ASOs. Indeed, for use in vivo, the oligonucleotide may be stabilised. A "stabilised" oligonucleotide refers to an oligonucleotide that is relatively resistant to in vivo degradation (e.g. via an exo- or endo-nuclease). Stabilisation can be a function of length or secondary structure. In particular, oligonucleotide stabilisation can be accomplished via phosphate backbone modifications.

In a particular embodiment, the target site blocker according to the invention is a LNA oligonucleotide. As used herein, the term "LNA" (Locked Nucleic Acid) (or "LNA oligonucleotide") refers to an oligonucleotide containing one or more bicyclic, tricyclic or polycyclic nucleoside analogues also referred to as LNA nucleotides and LNA analogue nucleotides".

### Nucleic Acid

The nucleic acids of the present invention inhibit miRNA binding in Arginase 2. The sequence of a nucleic acid inhibitor of miRNA according to the invention is sufficiently complementary to a miRNA MRE within Arg2 to hybridize to the miRNA binding site under physiological conditions and inhibit the activity or function of the miRNA in the cells of a subject. For instance, in some embodiments, nucleic acid inhibitors such as the target site blockers of the invention comprise a sequence that is at least partially complementary to a miRNA MRE within Arg2, e.g. at least about 75%, 80%, 85%, 90%, 95%, 96%, 97%, 98%, or 99% complementary to the miRNA MRE within Arg2. The nucleic acid inhibitor, such as the target site blockers of the invention, can be substantially complementary to a miRNA MRE within Arg2, that is at least about 90%, 95%, 96%, 97%, 98%, or 99% complementary to the miRNA MRE within Arg2. The nucleic acid inhibitor, such as the target site blockers of the invention, can comprise a sequence that is 100% or fully complementary to a miRNA MRE within Arg2. It is understood that the sequence of the nucleic acid inhibitor, such as the target site blockers of the invention, is considered to be complementary to a miRNA MRE within Arg2 even if the nucleic acid sequence includes a modified nucleotide instead of a naturally-occurring nucleotide.

### About

The term "about" as used herein encompasses variations of +/- 10% and more preferably +/-5%, as such variations are appropriate for practicing the present invention.

### Expression

Expression means any functions and steps by which a gene's coded information is converted into structures present and operating in a cell.

### Functionally active

By functionally active is meant a nucleic acid wherein the administration of nucleic acids to a subject or expression of nucleic acids in a subject enhances Arginase 2 expression in macrophages. Further, functional activity may be indicated by the ability of a nucleic acid to enhance Arginase 2 expression sufficiently to maintain macrophages in an anti-inflammatory and tissue regenerative state.

### Fragment

A fragment can comprise at least 1, 2, 3, 4, 5, 6, 7, 8, 9, 10, 15, 20, 22, or 25contiguous nucleotides from the nucleic acids of the present invention and which is functionally active. Suitably, a fragment may be determined using, for example, various different lengths nucleic acid sequences and testing for functionality using luciferase reporter assays.

### Variant

By variant is meant a nucleotide sequence which is at least 70% homologous to the nucleic acids of the present invention, more preferably at least 80% homologous to the nucleic acids of the present invention, more preferably at least 90% homologous to the nucleic acids of the present invention, even more preferably at least 95% homologous to the nucleic acids of the present invention, even more preferably at least 96% homologous to the nucleic acids of the present invention, even more preferably at least 97% homologous to the nucleic acids of the present invention, and most preferably at least 98% homology with the nucleic acids of the present invention. A variant encompasses a nucleic acid sequence of the nucleic acids of the present invention, which includes substitution of nucleotides, especially a substitution(s) which is/are known for having a high probability of not leading to any significant modification of the biological activity of the nucleic acid. Variants and fragments thereof may be generated using suitable molecular biology methods as known in the art.

### Subject

As herein defined, a "subject" includes and encompasses mammals such as humans, primates and livestock animals (e.g. sheep, pigs, cattle, horses, donkeys); laboratory test animals such as mice, rabbits, rats and guinea pigs; and companion animals such as dogs and cats.

### Treatment / Therapy

The term "treatment" is used herein to refer to any regimen that can benefit a human or non-human animal. The treatment may be in respect of inflammatory, autoimmune or reparative diseases mediated by macrophages, such as, multiple sclerosis, rheumatoid arthritis, colitis or peripheral nerve repair after injury and the treatment may be prophylactic (preventative treatment). Treatment may include curative or alleviative effects. Reference herein to "therapeutic" and "prophylactic" treatment is to be considered in its broadest context. The term "therapeutic" does not necessarily imply that a subject is treated until total recovery. Similarly, "prophylactic" does not necessarily mean that the subject will not eventually contract a disease condition. Accordingly, therapeutic and/or prophylactic treatment includes amelioration of the symptoms of a particular inflammatory, autoimmune or reparative condition mediated by macrophages or preventing or otherwise reducing the risk of developing a particular inflammatory, autoimmune or reparative condition. The term "prophylactic" may be considered as reducing the severity or the onset of a particular inflammatory or autoimmune condition. "Therapeutic" may also reduce the severity of an existing condition.

### Administration

The active ingredients used in the present invention in particular the target site blockers, as described herein, can be administered separately to the same subject, optionally sequentially, or can be co-administered simultaneously as a pharmaceutical or immunogenic composition. The pharmaceutical composition will generally comprise a suitable pharmaceutical excipient, diluent or carrier selected depending on the intended route of administration. The active ingredients can be administered to a patient in need of treatment via any suitable route. The precise dose will depend upon a number of factors, as is discussed below in more detail.

### Pharmaceutical Compositions

As described above, the present invention extends to a pharmaceutical composition for the treatment of inflammatory, autoimmune or reparative conditions mediated by macrophages, such as multiple sclerosis, rheumatoid arthritis, colitis or peripheral nerve repair after injury.

Pharmaceutical compositions according to the present invention, and for use in accordance with the present invention, may comprise, in addition to an active ingredient, a pharmaceutically acceptable excipient, carrier, buffer stabiliser or other materials well known to those skilled in the art. Such materials should be non-toxic and should not interfere with the efficacy of the active ingredient. The precise nature of the carrier or other material will depend on the route of administration, which may be, for example, oral, intravenous, intranasal or via oral or nasal inhalation. For example the target site blockers of the present invention can be encapsulated or complexed in biocompatible nanoparticles, such as, poly(lactic-co-glycolic acid) nanoparticles (PLGA-NPs), for specific drug delivery to macrophages. Other biocompatible nanoparticles can also be used, for example, a polymer-based nanoparticle such as one selected from the group comprising or consisting of Polyethylenimine (PEI) or its copolymers, Poly-L-lysine (PLL) or its copolymers, Polyethylene glycol (PEG) or its copolymers, Polycaprolactone, chitosan, Acetalated dextran or Star-Shaped Poly(l-lysine) Polypeptides. The biocompatible nanoparticle can be a liposome-based nanoparticle, such as Polyethylene glycol (PEG)-liposome or an inorganic nanoparticle such as one selected from the group comprising or consisting of gold, inorganic-organic hybrid or silica. The formulation may be a liquid, for example, a physiologic salt solution containing non-phosphate buffer at pH 6.8-7.6, or a lyophilised or freeze-dried powder.

### Dose

The composition is preferably administered to an individual in a "therapeutically effective amount" or a "desired amount", this being sufficient to show benefit to the individual. As defined herein, the term an "effective amount" means an amount necessary to at least partly obtain the desired response, or to delay the onset or inhibit progression or halt altogether the onset or progression of a particular condition being treated. The amount varies depending upon the health and physical condition of the subject being treated, the taxonomic group of the subject being treated, the degree of protection desired, the formulation of the composition, the assessment of the medical situation and other relevant factors. It is expected that the amount will fall in a relatively broad range, which may be determined through routine trials. Prescription of treatment, e.g. decisions on dosage etc., is ultimately within the responsibility and at the discretion of general practitioners, physicians or other medical doctors, and typically takes account of the disorder to be treated, the condition of the individual patient, the site of delivery, the method of administration and other factors known to practitioners. The optimal dose can be determined by physicians based on a number of parameters including, for example, age, sex, weight, severity of the condition being treated, the active ingredient being administered and the route of administration. A broad range of doses may be applicable. Dosage regimes may be adjusted to provide the optimum therapeutic response and reduce side effects. For example, several divided doses may be administered daily, weekly, monthly or other suitable time intervals or the dose may be proportionally reduced as indicated by the exigencies of the situation.

Unless otherwise defined, all technical and scientific terms used herein have the meaning commonly understood by a person who is skilled in the art in the field of the present invention.

### Conditions mediated by macrophages

Conditions mediated by macrophages include any disease or disorder wherein macrophages play a critical role. Macrophages play a critical role in the initial and progressive stages of both acute and chronic inflammatory, autoimmune, neurological and reparative diseases or disorders.

### Inflammatory conditions

The term "inflammatory conditions" as used herein is understood to mean any disease or disorder characterised by inflammation. Inflammatory conditions includes acute inflammatory conditions, chronic inflammatory conditions and reparative inflammatory conditions. Inflammation refers to a biological response to stimuli interpreted by the body to have a potentially harmful effect. While after injury or in certain conditions inflammation is a normal, healthy response, inflammatory disorders that result in the immune system attacking the body's own cells or tissues may cause abnormal inflammation, which results in chronic pain, redness, swelling, stiffness, and damage to normal tissues. Non-limiting examples of inflammatory conditions include multiple sclerosis, rheumatoid arthritis, colitis, allergy, asthma, pneumonia, autoimmune diseases, coeliac disease, glomerulonephritis, hepatitis, inflammatory bowel disease, preperfusion injury and transplant rejection.

The term "inflammatory conditions mediated by macrophages" as used herein is understood to mean any inflammatory disease or disorder where macrophages play a critical role and in particular where mediators of inflammation are induced by macrophages. Most of these cases are proinflammatory and pathogenic for disease progression, once activated macrophages actively secrete and cause an imbalance of cytokines, chemokines, and mediators of inflammation. Examples of inflammatory conditions mediated by macrophages include sepsis-related multiple organ dysfunction/multiple organ failure, microbial infection, acute brain/lung/hepatic/renal disorders, neurodegenerative disorders, tumorigenesis, osteoporosis/osteonecrosis, cardiovascular diseases, metabolic diseases, and autoimmune diseases. Specific examples of inflammatory conditions mediated by macrophages include multiple sclerosis, rheumatoid arthritis, colitis, pneumonia, Acute Respiratory Distress Syndrome (ARDS) including viral induced ARDS such as Covid-19 induced ARDS. Specific examples of neurodegenerative disorders include amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease.

### Autoimmune Condition

The term "autoimmune condition" as used herein is understood to mean any disease or condition which is caused by a body's tissues being attacked by its own immune system.

The term "autoimmune conditions mediated by macrophages" as used herein is understood to mean any autoimmune disease or disorder where macrophages play a critical role. In the target organs of autoimmune diseases, macrophages have dual functions in both the induction and suppression of autoimmune responses, which are mediated by production of various cytokines and chemokines, or by interaction with other immune cells. Examples of such autoimmune conditions mediated by macrophages include systemic lupus erythematosus, multiple sclerosis, rheumatoid arthritis, and Sjögren's syndrome.

### Reparative Condition

Reparative medicine is often used to denote the replacement, repair, or functional enhancement of tissues and organs.

The term "reparative conditions mediated by macrophages" as used herein is understood to mean any reparative disease of disorder wherein macrophages play a critical role. Macrophages play a critical role in reparative conditions such as peripheral nerve repair after injury.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

The present invention will now be exemplified with reference to the following non-limiting figures and examples which are provided for the purpose of illustration and are not intended to be construed as being limiting on the present invention. Other embodiments of this invention will be apparent to those of ordinary skill in the art in view of this description.

### Brief description of the Figures

Figure 1 shows the mechanism of action of a target site blocker (TSB).
Figure 2 demonstrates the effect of Arg2-TSB (SEQ ID NO:14) on miR-155-mediated repression of Arginase 2.
Figure 3 demonstrates the characterisation of size, surface charge and morphology of TSB-PLGA nanoparticles (NPs).
Figure 4 demonstrates the effect of Arg2-TSB (SEQ ID NO:14) transfection and TSB-PLGA nanoparticles (NPs) on viability and toxicity of Raw 264.7 cells and primary bone marrow-derived macrophages (BMDM).
Figure 5 demonstrates the effect of Arg2-TSB (SEQ ID NO:14) transfection and TSB-PLGA nanoparticles on release of the pro-inflammatory mediators (A) nitric oxide, (B) IL-6, (C) TNF-α and (D) IL-1β from Raws 264.7 cells.
Figure 6 demonstrates the effect of Arg2-TSB (SEQ ID NO:14) transfection and PLGA-TSBs on release of the pro-inflammatory mediators (A) nitric oxide, (B) IL-6, (C) TNF-α and (D) IL-1β from BMDM.

### Experimental Data

### Material and methods

### Cell culture and treatments

Raw 264.7 murine macrophage cell line was obtained from ATCC and cultured in Dulbecco's Modified Eagle's Medium (Sigma-D5796) supplemented with 10% heat-inactivated Fetal Bovine Serum (FBS) (Sigma-F9665) and 1% Penicillin-Streptomycin (pen/strep, 100 U/ml) (Sigma-P4333). Cells were routinely tested to be Mycoplasma negative. Cells were passaged twice a week (1:10) in T75 flasks. All experiments were carried out in early passage numbers, with passage number not exceeding 15 at most.

Bone marrow was isolated from wild-type (WT) C57BL6/J mice 6-12 weeks old adult female littermates. Mice were euthanized in a CO₂ chamber and death was confirmed by cervical dislocation. Femurs and tibias were isolated in sterile conditions, and the bone marrow was flushed out using Dulbecco's Phosphate Buffered Saline (DPBS). Marrow was spun and incubated with red blood cell (RBC) lysis buffer (Sigma) to remove red blood cells. A single cell suspension was prepared by passing the cells through a 70 µm cell strainer (Corning). They were then plated in 10 cm petri dishes in complete DMEM supplemented with 10% heat-inactivated FBS and 1% pen/strep. 20% L929 cells supernatant was also added to the culture to induce bone-marrow derived macrophages (BMDM) differentiation, after which cells were incubated for 6 days. In experiments, BMDM were seeded and stimulated in complete DMEM supplemented with 10% L929 cell supernatant.

L929 murine fibroblast cell line was obtained by ATCC and maintained in RPMI medium supplemented with 10% FBS and 1% pen/strep. L929 cells supernatant was generated from 20x10⁶ L929 cells plated in 40 ml of complete RPMI-1640 in T175 flasks for 10 days after which the media was filtered and frozen at -20ºC until use.

All cells were incubated in 37ºC with 5% CO₂ levels. Cell viability was determined using Trypan Blue and counted with a haemocytometer.

Fresh media was added to the cells before stimulation experiments. LPS (Sigma E.coli 0111:B4) was diluted from stock concentration of 1 mg/ml in complete DMEM, and used at a final concentration of 100 ng/ml. Cells were typically stimulated for 24 hours before conducting further assays.

### Experiment 1: The effect of Arg2 target site blocker on miR-155-mediated repression of Arginase 2

Figure 1 shows the mechanism of action of a target site blocker. Figure 1A shows a microRNA (for example miR-155, in red) which binds its target mRNA (for example *Arg2,* in green) through sequence-specific miRNA responsive elements (MREs, in purple) within the 3'UTR of Arg2, impeding its translation and leading to low quantity of the protein product. Figure 1B shows target site blockers (TSBs, in yellow) which are antisense oligonucleotides designed to effectively compete with endogenous miRNA by hybridizing to the same MRE. As a result TSBs will prevent endogenous miRNAs from binding to their MREs thereby increasing the expression of the protein encoded by the targeted mRNA and restoring its physiological levels.

The present inventors designed a target site blocker to compete with miR-155-5p (now on referred as miR-155), binding to its specific site within the murine Arg2 3'UTR (MRE at position 30-37) and its sequence is GTAATGCTGTTGTGAA (SEQ ID NO: 14). A scrambled TSB (i.e. not targeting anywhere in the genome, sequence ACGTCTATACGCCCA (SEQ ID NO:15)) was used as negative control (NC) in all experiments.

The full length murine Arg2 3'UTR luciferase plasmid was amplified using Q5 High-Fidelity DNA Polymerase (NEB) and inserted into XhoI-digested pmirGLO vector (Promega) using the GenBuilder Cloning Kit (Genscript). Plasmids were isolated from bacterial cultures with the Plasmid Midi Kit (Qiagen). In order to prove the specificity of Arg2-TSB (SEQ ID NO:14) for that particular binding site, a mutagenesis reaction was performed to disrupt its MRE at position 30-37 within the 3'UTR region using QuikChange Lightning Site-Directed Mutagenesis Kit (Agilent) using the wt plasmid (i.e. pmir_Arg2_wt) as template. Presence of the mutation in the mutant plasmid (i.e. pmir_Arg2_mut) was subsequently checked by screening with allele-specific oligonucleotide PCR (ASO-PCR) and sequencing. The sequence of cloning, mutagenesis and sequencing primers is reported in Table 2.

**Table 2: Primers names and sequences employed in this study.**

| **Name** | **Sequence** |
|---|---|
| **Cloning primers** | |
| Arg2_clon_F | Aacgagctcgctagcctcgaggaaatactgtactctggcac (SEQ ID NO:16) |
| Arg2_clon_R | Caggtcgactctagactcgagtatgatatactaaggtaataaatg (SEQ ID NO:17) |
| **Mutagenesis primers** | |
| Arg2_TSB_MUT_F | ctctggcacctttcacaacagc**TAAT**cagagttgcaaggcattcgaag (SEQ ID NO:18) |
| Arg2_TSB_MUT_R | cttcgaatgccttgcaactctg**ATTA**gctgttgtgaaaggtgccagag (SEQ ID NO:19) |
| **ASO-PCR primers** | |
| ASO_Arg2_wt | cacctttcacaacagc**ATTA** (SEQ ID NO:20) |
| ASO_Arg2_mut | cacctttcacaacagc**TAAT** (SEQ ID NO:21) |
| **Sequencing primers** | |
| pmir_seq_F | Gtggtgttgtgttcgtggac (SEQ ID NO:22) |
| pmir_seq_R | Cagccaactcagcttccttt (SEQ ID NO:23) |

| | |
|---|---|
| Mutagenesis primers: the mutant nucleotides are reported in capital letter, bold. ASO-PCR primers: Allele-Specific Oligonucleotide primers (wild type and mutant nucleotides in capital letter, bold) were designed to screen mutant from non-mutant colonies after mutagenesis. ASO-forward primers were used in combination with pmir_seq_R. Sequencing primers: pmir_seq_F and pmir_seq_R primers were designed on the plasmid sequence and they were employed for post-cloning screening and sequencing check. | |

In the luciferase assay experiments, Raw 264.7 cells were seeded in a 96-wells plate at a final density of 80,000 cells/well and incubated for 24 hours. Cells were then co-transfected with 100 ng of pmir_Arg2_wt or pmir_Arg2_mut and 100 nM of Arg2-TSB (SEQ ID NO:14) or NC-TSB (SEQ ID NO:15). Transfection mixes were prepared in serum free DMEM using TransIT-X2^{®} Transfection Reagent (Myrus). Luciferase activity was assessed at 24 hours after transfection using Dual-Luciferase Reporter Assay (Promega) according to the manufacturer's instructions. RLU (relative luciferase units) expressed as mean value of the firefly luciferase/Renilla luciferase ratio of at least three independent experiments performed in triplicate were used for statistical analyses.

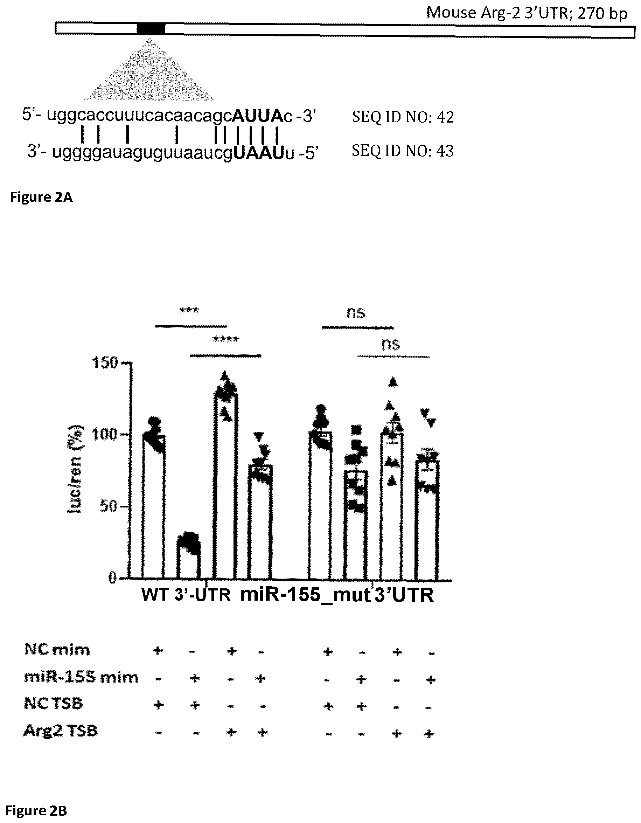

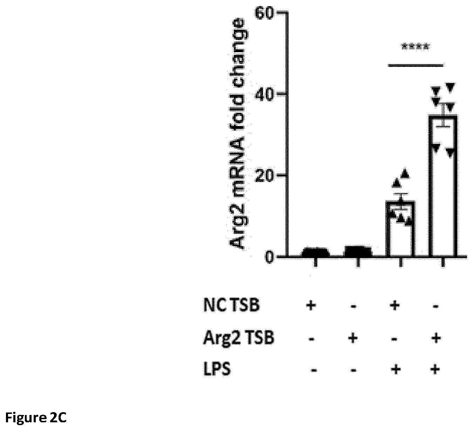

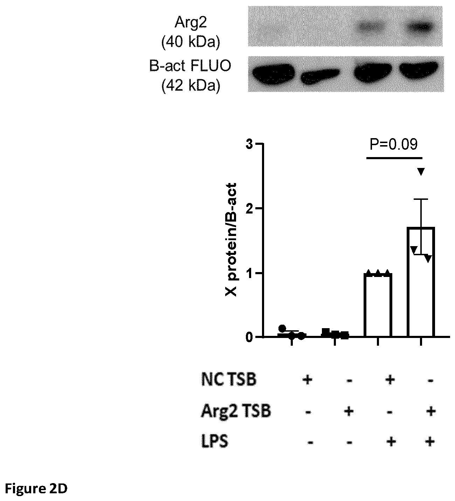

Figure 2 demonstrates the effect of Arg2-TSB (SEQ ID NO:14) on miR-155-mediated repression of Arginase 2. Arg2-TSB (SEQ ID NO:14) effectively blocks miR-155-mediated repression of Arginase-2 in luciferase assay, qRT-PCR and western blot in Raw 264.7 murine macrophage cell line. Figure 2A shows a visual map of the miRNA responsive element (MRE) of miR-155 within the Arg2 3'UTR at position 30-37. Figure 2B shows a luciferase assay following Arg2 (100 nM) co-transfection alone or in competition with miR-155 mimic (25 nM) in WT or miR-155-mutated plasmids (n=3, in triplicates); while Arg2-TSB (SEQ ID NO:14) is able to rescue the miR-155 dependent inhibition of Arg2-luciferase expression in a WT plasmid (first four bars), this effect is lost in the mutant plasmid where the miR-155 binding site was disrupted. Figure 2C and Figure 2D show the effect of Arg2-TSB (SEQ IDNO:14) (100 nM) transfection on endogenous levels of Arg2 (C) mRNA (n=2, in triplicates) and (D) protein levels (n=3, in single). ***P<0.001; ****P<0.0001.

### Experiment 2: Arg2 gene and protein expression analysis

For Arg2 mRNA expression, Raw 264.7 cells were seeded in a 24-wells plate at a final density of 375000 cells/well and after 24 hours they were transfected with 100 nM of Arg2-TSB (SEQ ID NO:14) or NC-TSB (SEQ ID NO:15) in DMEM serum free medium and Lipofectamine 3000 transfection reagent (ThermoFisher Scientific). At 24 hours post-transfection, cells were stimulated with 100 ng/mL LPS as previously stated for further 24 h. Total RNA was then extracted using TriReagent, and equal quantities were reverse transcribed into cDNA using High Capacity cDNA reverse transcription kit (Applied Biosystems) following the manufacturer's protocol. qRT-PCR was performed on the 7900 HT and 7500 Real-Time PCR System. Primers for Arg2 (forward 5'-GGATCCAGAAGGTGATGGAA-3' (SEQ ID NO:24), reverse 5'-AGAGCTGACAGCAACCCTGT-3' (SEQ ID NO:25)) and two housekeeping genes (*Rplp0:* forward 5-GGACCGCCTGGTTCTCCTAT-3' (SEQ ID NO:26), reverse 5'-ACGATGTCACTCCAACGAGG-3' (SEQ ID NO:27); *Tbp*: forward 5'-GAATTGTACCGCAGCTTCAAAAT-3' (SEQ ID NO:28) and reverse 5'-CAGTTGTCCGTGGCTCTCTT-3' SEQ ID NO:29)) were obtained from Sigma. Expression of Arg2 relative to the housekeeping genes was determined using the 2^{(-ΔΔCt)} method.

For Arg2 protein expression, Raw 264.7 cells were seeded in a 6-wells plate at a final density of 1.25x10⁶ cells/well and after 24 hours they were transfected with 100 nM of Arg2-TSB (SEQ ID NO:14) or NC-TSB (SEQ ID NO:15) in DMEM serum free medium and Lipofectamine 3000 transfection reagent (ThermoFisher Scientific). At 48 hours post-transfection, cells were stimulated with 100 ng/mL LPS as previously stated for further 24 h. Total protein was then extracted using low-stringency lysis buffer (50 mM HEPES (pH 7.5), 100 mM NaCl, 10% glycerol (v/v), 0.5% Nonidet P-40 (v/v), 1 mM EDTA, 1 mM sodium orthovanadate, 0.1 mM PMSF, 1 mg/ml aprotinin, and 1 mg/ml leupeptin). The resulting suspension was centrifuged at 12,000 rpm for 20 min at 4°C, and supernatants were collected and used for SDS-PAGE. Protein samples were normalised by BCA protein assay (Pierce), and denatured by the addition of 4X SDS sample buffer containing 0.2 M DTT and heated at 95°C for 10 min. Equal volumes of whole-cell lysates from were separated on 4-12% Bis-Tris acrylamide gels (Thermo Fisher Scientific), transferred to polyvinylidene difluoride membranes (Roche), and probed with mouse anti-Arg2 (1:1000, Invitrogen, Cat# MA5-27815) or anti-actin antibodies. Goat anti-mouse IgG, HRP-linked antibody (1:2500, Jackson Immunoresearch, Cat# 115-035-003) was used as a secondary antibody for one hour at RT for both Arg2 and β-actin antibodies. Detection was achieved using 20X LumiGLO^{®} Reagent and 20X Peroxide (Cell Signaling Technology, Cat# 7003) at the Amersham imager. For quantitative analysis, the signal

### Experiment 3: Cytokine and nitric oxide measurements

For cytokine measurements, cells were stimulated with LPS as indicated and supernatants removed and analysed for mouse IL-6, TNFα, and IL-1β using Enzyme-linked Immunosorbent Assay (ELISA) (DuoSet, R&D, respectively Cat# DY406, DY410 and DY401) according to manufacturers' instructions. NO production was measured from the same supernatants using the Griess Reagent (Sigma) by addition of reagent to sample in a 1:1 ratio. Absorbance in culture media was detected by a plate reader at 540 nm and compared against a standard curve.

### Experiment 4: Polylactide-co-glycolic acid nanoparticles (PLGA NPs) preparation

Arg2-TSB (SEQ ID NO:14) and NC-TSB (SEQ ID NO:15) (Qiagen) were encapsulated in DOTAP/PLGA NPs using the double emulsion solvent evaporation (DESE) method as previously described. To improve encapsulation efficiency TSBs were condensed with a cationic lipid DOTAP at an N/P (defined as the molar ratio of amine to phosphate groups) ratio of 4:1. Briefly, CFTR-specific LNAs were diluted in 200 µL of RNA-free water and DOTAP was dissolved in 200 µL of Tert-butanol. The TSB solution was added dropwise to the lipid mixture, mixed, and lyophilized overnight. 50 mg of PLGA Resomer^{®} RG 502 H (Sigma Cat# 719897) was dissolved in 2.5 mL of chloroform and briefly sonicated. Lyophilized TSB/DOTAP was resuspended in RNase-free water, added to the PLGA solution dropwise with a glass Pasteur pipette and sonicated for a total of 3 bursts of 5 s in continuous pulses mode at 70% amplitude to form the primary water-in-oil emulsion. The primary emulsion was added dropwise to a 2% poly(vinyl alcohol) (PVA) solution and sonicated on ice for 10 min in continuous pulses mode at 70% amplitude to form a secondary water-in-oil-in-water emulsion and then added to 2% PVA. The emulsion was mechanically stirred in the fume hood over night to allow the solvent to evaporate and allow NPs formation. NPs were then collected by centrifugation at 20,000 g for 15 min at 4ºC and washed three times with NaCl 1.13% in deionised water to remove residual PVA. Following this, TSB-PLGA NPs were resuspended in RNase-free water and freeze-dried for 24 h.

### Experiment 5: PLGA NPs characterisation and morphology

Size and zeta-potential of the TSB-PLGA NPs were measured by dynamic light scattering and by Laser Doppler Electrophoresis (LDE), respectively, on a Zetasizer Nano Series (Malvern Instruments). Measurements were made at 25°C. PLGA NPs were prepared at a concentration of 0.5 mg/ml and 1ml was used for measurement in the instrument. At least three independent batches of NPs, each prepared in triplicate, were used to determine the size distributions and the surface charge of the TSB-PLGA NPs.

Nanoparticles were visualised by transmission electron microscopy (TEM) in order to further confirm size and determine the morphology. Briefly, TSB-PLGA NPs were prepared at a concentration of 1 mg/ml in deionised water. 5µL of NPs suspension was placed on a Silicon Monoxide/Formvar coated grid (Mason technologies). Samples were allowed to air dry for approximately 10-15 min before being negative stained with 2% uranyl acetate alternative (URA) solution. Excess stain was removed using filter paper and the grids allowed to air dry fully before analysis. Imaging was performed using a Hitachi H-7650 Transmission Electron Microscope (Hitachi High Technologies, Berkshire, UK) at 120 kV.

### Experiment 6: TSB-PLGA NPs effect on macrophage viability and polarisation

Raw 264.7 cells and BMDM were seeded in 96-well plates at a final density of 80000 and 40000 cells/well, respectively, and incubated for 24 hours. Cells were then transfected with Arg2-TSB PLGA NPs or NC-TSB PLGA NPs resuspended in serum-free DMEM at a final concentration of 3mg/ml. In parallel, cells were also transfected with naked Arg2-TSB (SEQ ID NO:14) and NC-TSB (SEQ ID NO:15) using Lipofectamine 3000 transfection reagent in serum-free DMEM. At 24 hours post-transfection, cells were stimulated with 100 ng/mL LPS as previously stated for further 24 h. Supernatants were collected and used for NO and cytokines measurements using Greiss assay and ELISA respectively, as previously described. The impact of PLGA NPs on cell viability was assessed using CellTiter 96^{®} AQueous One Solution Cell Proliferation MTS Assay (Promega). In order to check the cytotoxicity of TSB-PLGA NPs, supernatants from control wells (i.e. from unstimulated cells) were also employed to measure lactate dehydrogenase (LDH) release from dying cells using CytoTox 96 NonRadioactive Cytotoxicity Assay (Promega).

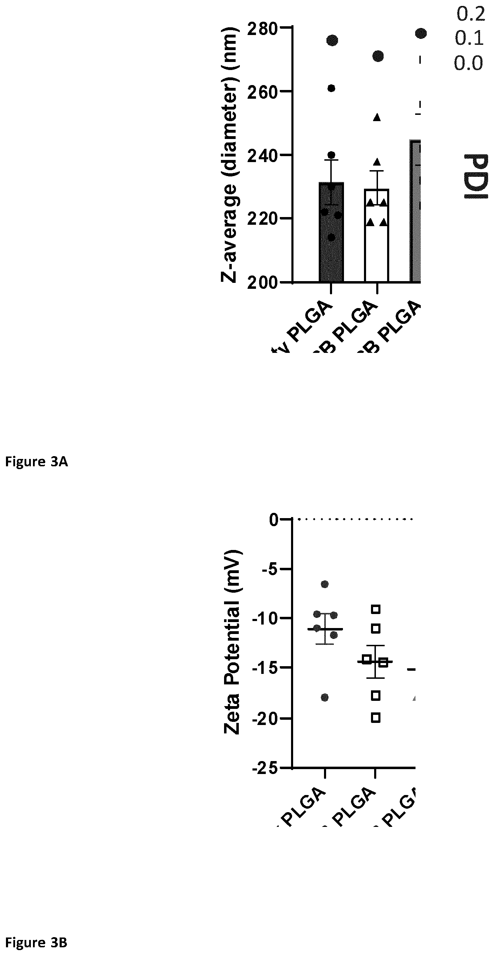

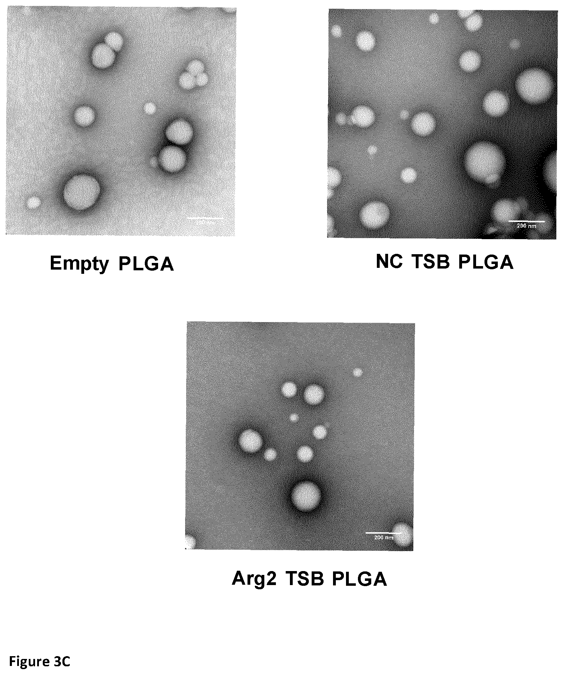

Figure 3 demonstrates the characterisation of size, surface charge and morphology of TSBs-PLGA nanoparticles (NPs). Figures 3 and 3B show the physicochemical characterisation of TSBs-PLGA NPs using the Zetasizer system for measuring (A) size, poly-dispersity index (PDI) and (B) surface charge. Figure 3C shows representative images of TSBs-PLGA NPs stained with UAR (Uranyl Acetate Replacement Stain) using transmission electron microscopy (TEM).

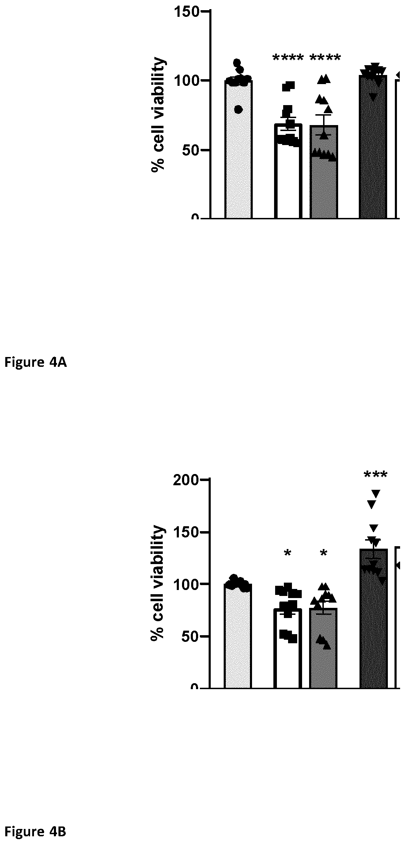

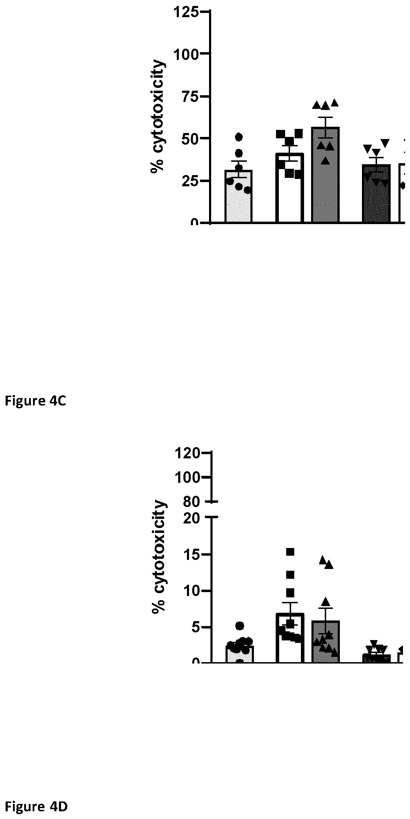

Figure 4 demonstrates the effect of Arg2-TSB (SEQ ID NO:14) transfection (with lipofectamine 3000 transfection reagent, second and third bars) and TSBs-PLGA nanoparticles (NPs) (forth, fifth and sixth bars) on Raw 264.7 cells (A) viability (3-(4,5-dimethylthiazol-2-yl)-5-(3-carboxymethoxyphenyl)-2-(4-sulfophenyl)-2H-tetrazolium = MTS assay, n=4, in triplicate) and (C) toxicity (lactate dehydrogenase = LDH assay, n=2, in triplicate). Similar results were obtained for primary bone marrow-derived macrophages (BMDM) (B) viability and (D) cytotoxicity.

Overall, lipofectamine transfection reagent showed significant reduction of cell viability and lead to the highest release of LDH in both Raw 264.7 and BMDM cells. PLGA NPs did not decrease cell viability and were not toxic compared to untransfected cells. Triton-X is used as positive control of cell death.

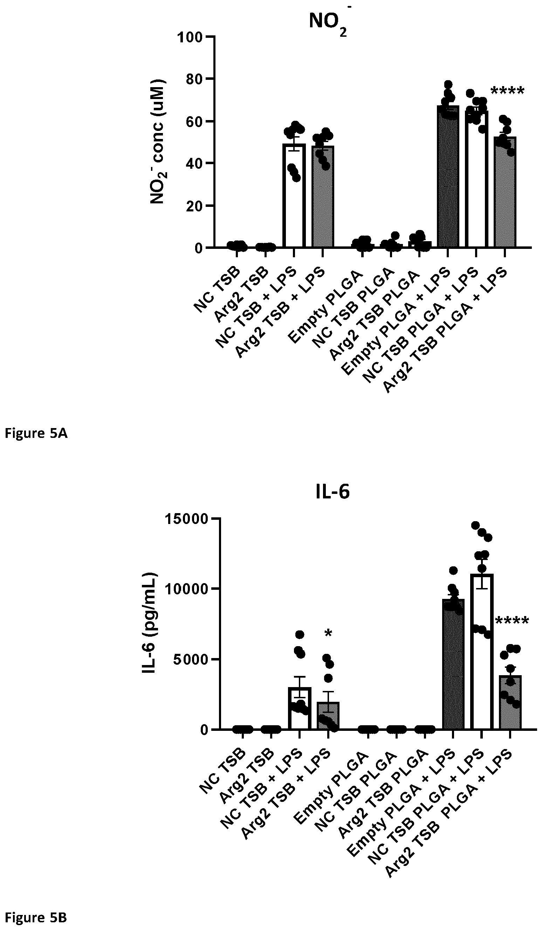

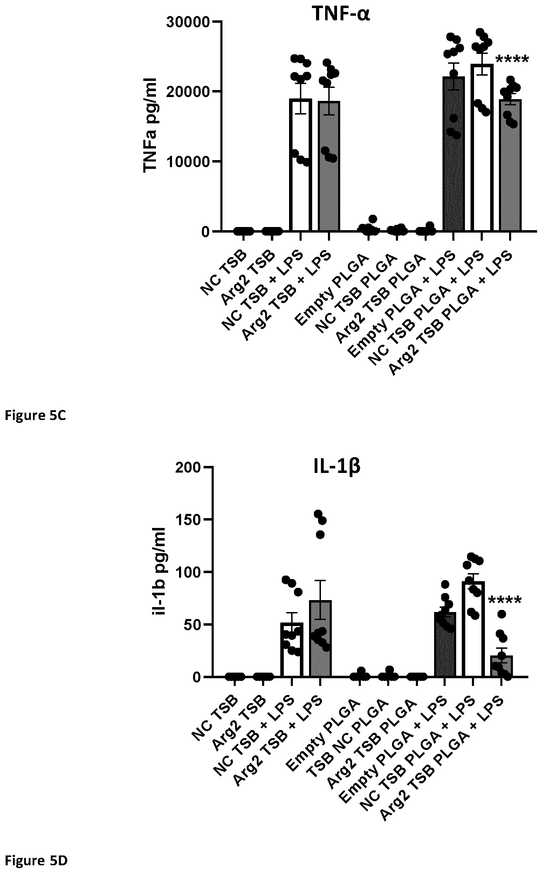

Figure 5 demonstrates the effect of Arg2-TSB (SEQ ID NO:14) transfection (with lipofectamine 3000 reagent, first four bars) and TSBs-PLGA (last six bars) on release of (A) nitric oxide, (B) IL-6, (C) TNF-α and (D) IL-1β from Raws 264.7 cells (n=3, in triplicate). ****P<0.0001. Arg2-TSB (SEQ IDNO:13) significantly reduced IL-6 release, but not NO, TNF-α or IL-1β when transfected with lipofectamine 3000. However, when encapsulated in PLGA NPs and therefore protected from lysosomal and endocytic degradation, Arg2-TSB-PLGA NP significantly reduces the secretion of all pro-inflammatory mediators in Raw 264.7 cells.

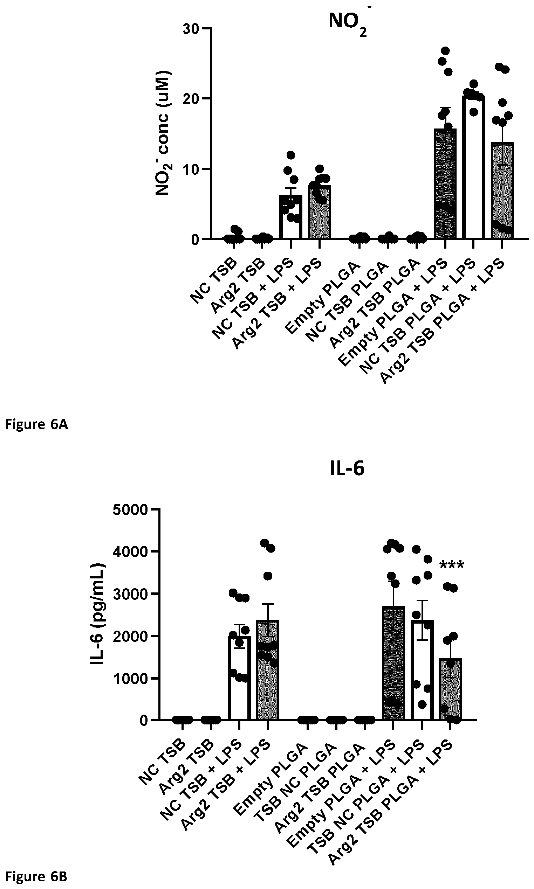

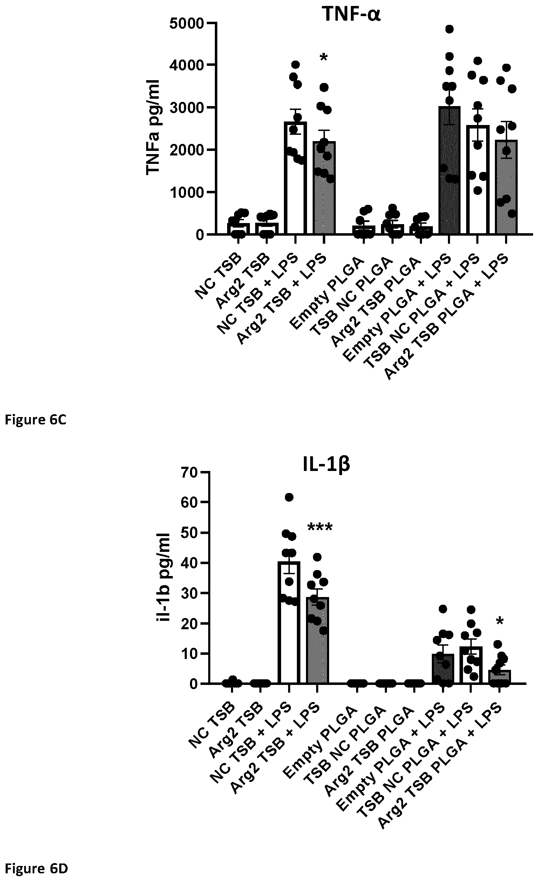

Figure 6 demonstrates the effect of Arg2-TSB (SEQ ID NO:14) transfection (with lipofectamine 3000 reagent, first four bars) and TSBs-PLGA (last six bars) on release of (A) nitric oxide, (B) IL-6, (C) TNF-α and (D) IL-1β from BMDM (n=3, in triplicate). *P<0.05,***P<0.001. The effect of Arg2-TSB (SEQ ID NO:14) on BMDM is similar to what we observed in Raws, however the cytokines mostly affected by Arg2-TSB (both transfected with lipofectamine 3000 or encapsulated into PLGA NPs) is Il-1β. NO production is not affected by Arg2-TSB (SEQ ID NO:14) in BMDM, while the effect on IL-6 and TNF-α levels changed depending on the type of transfection. Overall, higher variability was observed in BMDM due to biological differences between mice.

Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. A target site blocker for enhancing Arginase 2 expression in macrophages wherein the target site blocker is specific to miRNA binding sites in Arginase 2.

2. The target site blocker of claim 1, wherein the target site blocker is specific to miRNA binding sites in the 3'untranslated region of Arginase 2.

3. The target site blocker of claim 2, wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites.

4. The target site blocker of any preceding claim, wherein the target site blocker is specific to a miR-155 binding site in Arginase 2.

5. The target site blocker of any of claims 1 to 3, wherein the target site blocker is a nucleic acid molecule comprising or consisting of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof, or a combination thereof.

6. The target site blocker of any preceding claim, wherein the target site blocker is encapsulated or complexed in a biocompatible particle/ nanoparticle; optionally wherein the biocompatible nanoparticle is a poly(lactic-co-glycolic acid) nanoparticle (PLGA-NP).

7. A target site blocker for enhancing Arginase 2 expression in macrophages, wherein the target site blocker is specific to miRNA binding sites in Arginase 2, for use in the treatment and/or prophylaxis of conditions mediated by macrophages.

8. The target site blocker for use in the treatment and/or prophylaxis of conditions mediated by macrophages of claim 7, wherein the target site blocker is specific to miRNA binding sites in the 3'untranslated region of Arginase 2, optionally wherein the miRNA binding site is selected from the group comprising or consisting of miR-155, miR-1299, miR-199a, miR-199b, miR-10a, miR-10b, miR-1278, miR-570, miR-1252, miR-3202, let-7a, let-7b, let-7c, let-7e, let-7f, let-7g, let-7i, miR-98, miR-1294 or miR-9 binding sites or combinations thereof.

9. The target site blocker for use in the treatment and/or prophylaxis of conditions mediated by macrophages of any of claims 7 or 8, wherein the target site blocker is specific to a miR-155 binding site in Arginase 2.

10. The target site blocker for use in the treatment and/or prophylaxis of conditions mediated by macrophages of any of claims 7 to 9, wherein, the target site blocker is a nucleic acid molecule comprising or consisting of the nucleotide sequence of SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or a fragment or variant thereof, or a combination thereof.

11. The target site blocker for use in the treatment and/or prophylaxis of conditions mediated by macrophages of any of claims 7 to 10, wherein the conditions mediated by macrophages are inflammatory conditions, autoimmune conditions, neurological conditions or reparative conditions.

12. The target site blocker for use in the treatment and/or prophylaxis of conditions mediated by macrophages of any of claims 7 to 11, wherein the inflammatory or autoimmune condition mediated by macrophages is multiple sclerosis, rheumatoid arthritis or colitis.

13. The target site blocker for use in the treatment and/or prophylaxis of conditions mediated by macrophages of any of claims 7 to 11, wherein the neurological condition mediated by macrophages is a neurodegenerative condition selected from the group comprising amyotrophic lateral sclerosis, Parkinson's disease, Huntington's disease, Alzheimer's disease, dementia, Traumatic brain injury, epilepsy and all versions of epilepsy (including, for example, FIRES, RAusmann etc), rare diseases such as Rett syndrome, leukocephalopathy, encephalopatmus, and Nasu-Hakola disease and wherein the inflammatory conditions can be a condition selected from pneumonia, acute respiratory distress syndrome and Covid-19 related acute respiratory distress syndrome.

14. The target site blocker of any preceding claim, wherein the target site blocker is encapsulated or complexed to a particle carrier, microparticle, implantable scaffold or biocompatible nanoparticle; optionally wherein the biocompatible nanoparticle is a poly(lactic-co-glycolic acid) nanoparticle (PLGA-NP).

15. A pharmaceutical composition comprising:
(i) a target site blocker for enhancing Arginase 2 expression in macrophages, wherein the target site blocker is specific to miRNA binding sites in Arginase 2;
(ii) a biocompatible carrier, optionally a biocompatible nanoparticle carrier;
optionally wherein the biocompatible nanoparticle carrier is a poly(lactic-co-glycolic acid) nanoparticle (PLGA-NP).
